# FASCICULE DE BREVET EUROPEEN

(11) **EP 0 850 644 B1**
(45) Date de publication et mention de la délivrance du brevet: **22.09.1999**
(21) Numéro de dépôt: 97403030.6
(22) Date de dépôt: 12.12.1997
(51) Int. Cl.: A61K 7/48, A61K 7/02, A61K 7/021, A61K 7/027

(54) **Composition de maquillage ou de soin ne migrant pas, contenant un organopolysiloxane solide élastomère et une phase grasse**
Abriebfeste Schmink- oder Körperpflegezusammensetzung mit einem festen elastomerischen Organopolysiloxane und einer Fettphase
Make up or personal care composition which does not rub off and which comprises a solid elastomeric organopolysiloxane and a fatty phase

(30) Priorité: 24.12.1996 FR 9615985
(43) Date de publication de la demande: 01.07.1998
(73) Titulaire: L'OREAL, 75008 Paris (FR)
(72) Inventeur: Bara, Isabelle, 75013 Paris (FR); Auguste, Frédéric, 94550 Chevilly Larue (FR)
(74) Mandataire: Lhoste, Catherine

(56) Documents cités:
- CHEMICAL ABSTRACTS, vol. 126, no. 8, 24 février 1997 Columbus, Ohio, US; abstract no. 108664, KURODA, AKIHIRO ET AL: "Sunscreens containing metal oxides, polyoxyalkylene-polysiloxanes, and elastomers or resin waxes" XP002042939 & JP 08 295 620 A (KANEBO LTD, JAPAN)
- CHEMICAL ABSTRACTS, vol. 124, no. 4, 22 janvier 1996 Columbus, Ohio, US; abstract no. 37392, NAGANUMA, MASAYUKI ET AL: "Makeup cosmetics containing powdered organopolysiloxane elastomers, nonporous spherical silica, and oils" XP002042940 & JP 07 258 028 A (SHISEIDO CO LTD, JAPAN)

## Description

La présente invention se rapporte à l'utilisation d'organopolysiloxane particulier dans une composition de soin et/ou de maquillage de la peau et/ou des lèvres des êtres humains, et en particulier dans un rouge à lèvres ou un fond de teint, sous forme de stick, de coupelle ou de crème contenant un organopolysiloxane.

Les compositions de rouge à lèvres et de fond de teint connues comprennent généralement des corps gras tels que des huiles, des composés pâteux et des cires, ainsi qu'une phase particulaire généralement composée de charges et de pigments.

Dans les rouges à lèvres classiques, les huiles confèrent une grande partie des propriétés de ces rouges, telles que la facilité d'application, le glissant, la brillance. En outre, la majeur partie de ces huiles et notamment les huiles végétales confèrent aux rouges des propriétés traitantes. Il en est de même pour les fonds de teint.

Malheureusement ces compositions de maquillage, lorsqu'elles sont appliquées sur la peau ou les lèvres, et notamment les pigments de ces compositions ont tendance à migrer, c'est-à-dire à se propager à l'intérieur des rides et des ridules de la peau qui entourent les lèvres et les yeux, entraînant un effet inesthétique. L'apparition de ces traces a tendance à écarter certaines femmes de l'utilisation de ce type de maquillage.

Il est connu du document 〈〈 Chemical Abstracts 〉〉 vol. 24, n°4 22/01/96, des compositions de maquillage non collantes contenant des élastomères organopolysiloxane, de la silice et des huiles.

La présente invention a pour objet l'utilisation d'organopolysiloxane particulier dans une composition de soin ou de maquillage permettant de remédier à ces inconvénients et permettant en particulier l'obtention d'un film ne migrant pas et présentant des propriétés cosmétiques comme notamment des propriétés de glissant, de non tiraillement et de non dessèchement des lèvres ou de la peau.

L'invention s'applique non seulement aux produits de maquillage des lèvres mais aussi aux produits de soin et/ou de traitement des lèvres, ainsi qu'aux produits de maquillage, de soin et/ou de traitement de la peau.

De façon plus précise, l'invention a pour objet l'utilisation d'un organopolysiloxane élastomérique solide au moins partiellement réticulé, comme gélifiant dans une composition de maquillage ou de soin, afin de diminuer la migration de ladite composition.

Par 〈〈 élastomérique 〉〉 on entend un matériau souple, déformable ayant des propriétés viscoélastiques et notamment la consistance d'une éponge ou d'une sphère souple.

L'invention a donc pour objet l'utilisation cosmétique d'un organopolysiloxane élastomérique solide au moins partiellement réticulé, comme gélifiant, dans une composition de maquillage ou de soin d'une zone de la peau ou des lèvres d'êtres humains afin de diminuer la migration de ladite composition autour de ladite de zone ou des lèvres.

Les organopolysiloxanes élastomériques de la composition de l'invention présentent un remarquable pouvoir gélifiant d'huile. Ils ne sont pas desséchants pour la peau et apportent de bonnes propriétés cosmétiques. Ces nouveaux élastomères conduisent à des compositions confortables à l'application, douces et non collantes au toucher. Cette douceur est due, d'une part à la texture des organopolysiloxanes et, d'autre part à leurs propriétés comparables à celles de microéponges piégeant les huiles. En outre, grâce au piégeage des huiles dans l'organopolyloxanes et, d'autre part à leurs propriétés comparables à celles de microéponges piégeant les huiles. En outre, grâce au piégeage des huiles dans l'organopolysiloxane, les compositions de l'invention ne migrent plus dans les ridules et les rides de la peau, même lorsqu'elles contiennent des huiles traitantes.

Les compositions de l'invention peuvent se présenter sous forme de pâte, de solide, de crème. Elles peuvent être une émulsion huile-dans-eau ou eau-dans-huile, un gel anhydre, solide ou souple.

Les organopolysiloxanes élastomériques conformes à l'invention sont partiellement ou totalement réticulés et de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel homogène, en présence de quantités de phase grasse plus élevées. La gélification de la phase grasse par ces élastomères peut être totale ou partielle.

Les élastomères de l'invention sont véhiculés généralement sous forme de gel constitué d'un organopolysiloxane élastomère de structure tridimensionnelle, incluant au moins une huile hydrocarbonée et/ou une huile siliconée.

Les organopolysiloxanes élastomériques selon l'invention peuvent être choisis parmi les polymères réticulés décrits dans la demande EP-A-0295886. Selon cette demande, ils sont obtenus par réaction d'addition et de réticulation, en présence d'un catalyseur du type platine, d'au moins :
- (a) un organopolysiloxane ayant au moins deux groupes alcényle inférieurs en C₂ à C₆ par molécule ; et
- (b) un organopolysiloxane ayant au moins deux atomes d'hydrogène liés à un atome de silicium par molécule.

Les organopolysiloxanes élastomériques selon l'invention peuvent aussi être choisis parmi ceux décrits dans le brevet US 5.266.321. Selon ce brevet, ils sont choisis notamment parmi :
- i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs R₂SiO sur les motifs RSiO_{1,5} allant de 1/1 à 30/1 ;
- ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

Les organopolysiloxanes de la composition de l'invention sont par exemple ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels déjà constitués (KSG15, KSG17, KSG16, KSG18 de Shin-Etsu, Gransil SR 5CYC gel, Gransil SR DMF 10 gel, Gransil SR DC556 gel, SF 1204 et JK 113 de General Electric. On peut aussi utiliser un mélange de ces produits commerciaux.

De façon préférentielle, l'organopolysiloxane est présent dans le mélange organo-polysiloxane/phase grasse sous forme d'un gel plus ou moins homogène à une concentration allant de 0,3 à 40 % du poids total de la composition. De préférence l'élastomère représente, en matière active, de 0,1 à 20% dans la composition et de préférence de 0,3 à 15%.

La phase grasse peut être quelconque ; elle contient des huiles (produits fluides à température ambiante) qui peuvent êtres des huiles siliconées, fluorées, fluorosiliconées, hydrocarbonées éventuellement partiellement siliconées. La meilleure gélification est réalisée avec les huiles siliconées ou partiellement siliconées, des huiles apolaires et peu polaires et quelques huiles polaires qui ne nuisent pas à la stabilité du système. Ces huiles sont notamment déterminées en fonction de leurs paramètres de solubilité définis dans l'espace de Hansen.

En particulier, la composition de l'invention comprend des huiles non volatiles classiquement utilisées dans le domaine d'application envisagé et des cires.

Comme huiles non volatiles utilisables dans l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R⁹COOR¹⁰ dans laquelle R⁹ représente le reste d'un acide gras supérieur comportant de 7 à 19 atomes de carbone et R¹⁰ représente une chaîne hydrocarbonée ramifiée contenant de 3 à 20 atomes de carbone comme par exemple l'huile de Purcellin ;
- les hydrocarbures linéaires ou ramifiés, d'origine minérale ou synthétique tels que les huiles de paraffine non volatiles et leurs dérivés, la vaseline, les polydécènes, le polyisobutène hydrogéné tel que le parléam;
- les esters et les éthers de synthèse comme le myristate d'isopropyle, des octanoates, décanoates ou ricinoléates d'alcools ou de polyalcools ;
- des alcools gras comme l'actyl dodécanol ou l'alcool oléique ;
- les huiles fluorées partiellement hydrocarbonées et/ou siliconées comme celle décrites dans le document JP-A-2-295912 ;
- les huiles siliconées comme les polyméthylsiloxanes non volatiles linéaires (PDMS), liquides ou pâteux à température ambiante, les phényl diméthicones, les phényl triméthicones et les polyméthylphénylsiloxanes ;
- leurs mélanges.

Les huiles représentent de 5 à 80% du poids total de la composition, de préférence de 20 à 70% et mieux de 30 à 50 %. En particulier, les huiles non volatiles représentent de 4 à 60% et mieux de 30 à 50% du poids total de la composition, les huiles volatiles représentant alors le complément.

Les cires des compositions de l'invention sont des cires hydrocarbonées, fluorées ou siliconées ou des mélanges de cires, qui peuvent être solides ou semi-solides (sous forme d'une pâte) à température ambiante. Ces cires peuvent être d'origine végétale, minérale, animale et/ou synthétique. En particulier, ces cires présentes une température de fusion supérieure à 25 °C et mieux supérieure à 45 °C.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Parmi les cires de silicone utilisables dans l'invention, on peut citer la béhénoxy diméthicone telle que celle vendue par Goldschmidt sous le nom Abil Wax 2440 ; la stéaryl diméthicone telle que celle vendue par Dow Corning sous le nom DC 2503 ; la cétyl diméthicone telle que celle vendue par Goldschmidt sous le nom Abil Wax 9814 ; la stéaryl méthicone telle que celle vendue par Goldschmidt sous le nom Abil Wax 9809 ; la C₂₄-C₂₈ alkyl diméthicone telle que celle vendue par Goldschmidt sous le nom Abil Wax 9810 ; la C₃₀-C₄₅ alkyl méthicone telle que celle vendue par Goldschmidt sous le nom Abil Wax 9811 ; la stéaroxy diméthicone telle que celle vendue par Goldschmidt sous le nom Abil Wax 2434 ; la béhénate diméthicone comme celle vendue par Rhône Poulenc sous le nom Myrasil Wax B.

Comme autres cires de silicone utilisables dans l'invention, on peut citer les copolymères d'alkyl diméthicones. Ces copolymères sont notamment ceux décrits dans les documents EP-A-527594, US-A-5 061 481, US-A- 5397 566, EP-A-527594. On peut aussi citer les cires de silicones modifiées par des chaînes fluorées comme celles décrites dans le document EP-A-661 042.

Comme autre cires utilisables dans l'invention, on peut citer les cires d'origine animale comme la lanoline, la cire d'abeille ; les cires végétales telles que la cire de Carnauba ou de Candellila ; les cires minérales, par exemple de paraffine, de lignite ou les cires microcristallines, la cérésine ou l'ozokérite ; les cires synthétiques comme les cires de polyéthylène.

La nature des cires et des huiles ainsi que leurs quantités peuvent être choisies de manière variée par l'homme du métier afin de préparer une composition ayant les propriétés cosmétiques, et notamment la consistance ou la texture, souhaitées.

En particulier, la présence d'une grande quantité de cires permet d'assurer une bonne résistance mécanique, notamment lorsque la composition se présente sous la forme d'un stick.

D'une manière générale, la composition peut comprendre de 1 à 50% du poids total de la composition de cire et de préférence de 10 à 30%.

La phase grasse peut, en plus des huiles et cires décrites précédemment, contenir une ou plusieurs huiles volatiles à température ambiante. Par huile volatile, on entend une huile susceptible de s'évaporer au contact de la peau ou des lèvres.

Ces huiles volatiles peuvent être des huiles hydrocarbonées, des huiles de silicones ou leurs mélanges. Les silicones volatiles sont par exemple des silicones comportant une structure silicone linéaire et des motifs à chaîne alkyle pendante et/ou en bout de structure silicone, ces chaînes alkyle étant linéaires ou ramifiées et comportant de 3 à 10 atomes de carbone. Les silicones volatiles à chaîne alkyle présentent notamment la formule (1) suivante :

R'(CH₃)₂Si-O-{-Si-(CH₃)R''-O-}ₙ-{-Si-(CH₃)₂-O-}ₘ-Si(CH₃)₂R''

où R' et R'' sont indépendamment H, méthyle ou une chaîne ayant de 3 à 10 atomes de carbone, n et m étant des entiers allant de 0 à 10 à condition que si R' est hydrogène ou méthyle, n est différent de 0 et R'' est une chaîne alkyle de 3 à 10 atomes de carbone. Comme silicones volatiles alkylées utilisables dans l'invention, on peut citer les alkyl heptaméthyltrisiloxanes avec un groupe alkyle en C₄, C₅, C₆, C₇ et C₈ comme par exemple l'hexyl heptaméthyltrisiloxane de formule : (CH₃)₃-Si-O-Si(CH₃)(C₆H₁₃)-O-Si(CH₃)₃ ; l'octyl heptaméthyltrisiloxane de formule : (CH₃)₃-Si-O-Si(CH₃)(C₈H₁₅)-O-Si(CH₃)₃ ; et leurs mélanges.

Comme silicones volatiles utilisables dans l'invention, on peut encore citer les polydiméthylsiloxanes à chaîne linéaire ayant de 2 à 6 atomes de silicium. Ces silicones satisfont à la formule (1) avec m valant 0, n valant de 0 à 6 et R' et R'' représentant simultanément CH₃ ou phényle. On peut par exemple citer les méthyl-polysiloxanes comme l'hexaméthyldisiloxane, les méthylphénylpolysiloxanes, les éthylpolysiloxanes, les éthylméthylpolysiloxanes, les éthylphénylpolysiloxanes, les hydroxyméthylpolysiloxanes et leurs mélanges.

Comme silicones volatiles, on peut aussi utiliser des silicones cycliques ayant de 3 à 7 motifs -R¹R²SiO-, avec R¹ et R² représentant indépendamment H, méthyle, éthyle ou phényle. A titre d'exemple, on peut citer l'octaméthylcyclopentasiloxane, le décaméthylcyclopentasiloxane ou leurs mélanges.

Comme huiles volatiles hydrocarbonées utilisables dans l'invention, on peut citer les isoparaffines en C₃ à C₂₀ comme l'isoparaffine en C₁₂ appelé isododécane et l'isoparaffine en C₁₆ comme l'isohexadécane.

La composition de l'invention peut encore comprendre tout additif usuellement utilisé dans le domaine concerné, tel que des colorants hydrosolubles ou liposolubles, des antioxydants, des huiles essentielles, des conservateurs, des actifs cosmétiques ou dermatologiques, des hydratants, des vitamines, des acides gras essentiels, des filtres solaires lipophiles, des polymères liposolubles notamment hydrocarbonés tels que les polyalkylènes. Ces additifs peuvent être présents dans la composition à raison de 0 à 20% du poids total de la composition et mieux de 0 à 10%.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée. En particulier, ces additifs ne devront pas nuire à l'homogénéité, la stabilité, le confort et à la 〈〈 non migration 〉〉 de la composition.

Les compositions selon l'invention peuvent se présenter notamment sous la forme de stick ou bâton, ou sous la forme de pâte souple ou coulée, voire sous la forme d'un liquide huileux gélifié ou d'une crème.

La composition selon l'invention peut se présenter sous la forme d'un produit coloré de maquillage de la peau, en particulier un fond de teint, un fard à joues ou à paupières, un anti-cernes ou de maquillage des lèvres comme un rouge à lèvres. Elle peut également se présenter sous forme non colorée, contenant éventuellement des actifs cosmétiques ou dermatologiques. Elles peuvent alors être utilisées comme base de soin pour les lèvres (baumes à lèvres, protégeant les lèvres du froid et/ou du soleil et/ou du vent) ou base fixante à appliquer sur un rouge à lèvres classique. La base fixante forme alors un film protecteur sur le film de rouge, qui en limite la migration.

La composition de l'invention peut également se présenter sous la forme d'une composition dermatologique ou de soin de la peau, ou sous forme d'une composition de protection solaire.

Bien entendu la composition de l'invention doit être cosmétiquement ou dermatologiquement acceptable, à savoir non toxique et susceptible d'être appliquée sur la peau ou les muqueuses (lèvres, intérieur des paupières) d'être humains.

De façon préférentielle, la composition de l'invention peut comprendre une phase particulaire, généralement présente à raison de 0 à 35 % du poids total de la composition, de préférence de 5 à 25 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la cire et la silicone volatile, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 25 % du poids de la composition finale, et de préférence à raison de 5 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium.

Les nacres peuvent être présentes dans la composition à raison de 0 à 20 % du poids total de la composition, de préférence à un taux élevé de l'ordre de 8 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 5 à 15 %. On peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

De façon plus spécifique, l'invention a porte sur un rouge à lèvres ou fond de teint anhydre sans migration, contenant l'organopolysiloxane au moins partiellement réticulé solide élastomérique, comme gélifiant, associé à une phase grasse renfermant au moins une huile, et au moins une cire, et une charge pigmentaire.

La composition selon l'invention peut être fabriquée par chauffage d'un ou plusieurs organopolysiloxanes élastomériques associés à une ou plusieurs huiles, une ou plusieurs cires, un ou plusieurs pigments, une ou plusieurs charges et/ou un ou plusieurs autres additifs à une température supérieure à la température la plus élevée de fusion des cires, puis coulage du mélange fondu dans un moule. Ce procédé permet d'obtenir une composition sous forme solide de stick ou de coupelle.

Elle peut aussi être obtenue par extrusion comme décrit dans la demande EP-A-667 146. Ce procédé consiste à malaxer la pâte (cires + huiles + additifs + pigments) pendant le refroidissement pour créer dans la masse des zones d'écrasement de la pâte à l'aide d'un broyeur à cylindres ou d'un extrudeur-mélangeur à vis. Ce procédé permet l'obtention d'une composition sous forme de pâte molle.

L'invention a encore pour objet l'utilisation d'un organopolysiloxane élastomérique solide au moins partiellement réticulé, comme gélifiant pour la fabrication d'une composition de soin ou de traitement d'une zone de la peau ou des lèvres d'êtres humains afin de diminuer la migration de ladite composition autour de ladite de zone ou des lèvres.

L'invention a encore pour objet un procédé pour limiter et/ou empêcher la migration d'une composition de maquillage ou de soin de la peau ou des lèvres, consistant à introduire dans la composition au moins un organopolysiloxane élastomérique solide au moins partiellement réticulé.

L'invention a encore pour objet un procédé de traitement cosmétique ou dermatologique de la peau ou des lèvres d'êtres humains, consistant à appliquer sur la peau ou les lèvres une composition cosmétique telle que définie ci-dessus.

L'invention est illustrée plus en détail dans l'exemple suivant. Les pourcentages sont donnés en poids.

| **Exemple 1** | |
|---|---|
| Cire de polyéthylène | 15,0 % |
| Organopolysiloxane réticulé à 60% dans un PDMS non volatil (KSG6) | 10,0 % |
| Phényl trimethicone (huile) | 25,0 % |
| Stearyl dimethicone (pâteux) | 30,0 % |
| Lanoline | 10,0 % |
| Pigments | 10,0 % |

Cette composition est obtenue en faisant gonfler l'organopolysiloxane dans les huiles à température ambiante. On empâte ensuite les pigments dans le gel obtenu, à 60°C, puis on broie le tout dans un broyeur tricylindre à température ambiante. On mélange le broyât à la cire et au pâteux, à 95°C, puis on coule dans un moule approprié. On obtient ainsi un stick de rouge à lèvres de texture agréable, s'étalant bien et ne migrant pas au cours du temps, après son application sur les lèvres. Le film obtenu est satiné.

La composition C1 de l'exemple 1 a été comparée avec une composition contre-exemple C2 contenant les mêmes ingrédients que ceux de la composition C1 mais pas d'organopolysiloxane. Les compositions C1 et C2 ont été appliquées par demi-lèvres par des esthéticiennes. La composition C1 à été jugée, par toutes les personnes testrices, comme migrant beaucoup moins que la composition C2, tout en présentant des propriétés cosmétiques similaires.

## Revendications

1. Utilisation cosmétique d'un organopolysiloxane solide élastomérique au moins partiellement réticulé, comme gélifiant, dans une composition de maquillage ou de soin d'une zone de la peau ou des lèvres d'êtres humains afin de diminuer la migration de ladite composition autour de ladite de zone ou des lèvres.

2. Utilisation d'un organopolysiloxane solide élastomérique au moins partiellement réticulé, comme gélifiant, pour la fabrication d'une composition de soin ou de traitement d'une zone de la peau ou des lèvres d'êtres humains afin de diminuer la migration de ladite composition autour de ladite de zone ou des lèvres.

3. Utilisation selon la revendication 1 ou 2, caractérisée en ce que l'organopolysiloxane élastomérique est obtenu par réaction d'addition et de réticulation, en présence d'un catalyseur, d'au moins :
(a) un organopolysiloxane ayant au moins deux groupes alcényle inférieurs en C₂ à C₆ par molécule;
(b) un organopolysiloxane ayant au moins deux atomes d'hydrogène liés à un atome de silicium par molécule.

4. Utilisation selon l'une quelconque des revendications précédentes, caractérisée en ce que l'organopolysiloxane est choisi parmi :
- i) les organopolysiloxanes comprenant des motifs R₂SiO et RSiO_{1,5} et éventuellement des motifs R₃SiO_{0.5} et/ou SiO₂ dans lesquels les radicaux R, indépendamment les uns des autres, représentent un hydrogène, un alkyle, un aryle, un groupe aliphatique insaturé, le rapport en poids des motifs R₂SiO sur les motifs RSiO_{1,5} allant de 1/1 à 30/1 ;
- ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

5. Utilisation selon l'une des revendications précédentes, caractérisée en ce que l'organopolysiloxane solide élastomérique est présent dans le mélange organopolysiloxane élastomérique/phase grasse pour former un gel homogène dans une concentration allant de 0,3 à 40 % en poids.

6. Utilisation selon l'une des revendications précédentes, caractérisée en ce que la composition comporte au moins une huile.

7. Utilisation selon la revendication 6, caractérisée en ce que l'huile est une huile non volatile.

8. Utilisation selon la revendication 6 ou 7, caractérisée en ce que l'huile est présente à raison de 5 à 80 %, de préférence 30 à 50 %, du poids total de la composition.

9. Utilisation selon l'une quelconque des revendications précédentes, caractérisée en ce que l'huile est choisie parmi les huiles hydrocarbonées d'origine animale, végétale ; les huiles de formule R⁹COOR¹⁰ dans laquelle R⁹ représente le reste d'un acide gras supérieur comportant de 7 à 19 atomes de carbone et R¹⁰ représente une chaîne hydrocarbonée ramifiée contenant de 3 à 20 atomes de carbone ; les hydrocarbures linéaires ou ramifiés d'origine minérale ou synthétique ; les alcools gras ; les esters et éthers de synthèse ; les polyméthylsiloxanes linéaires liquides ou pâteux à température ambiante ; les phényl diméthicones ; les phényl triméthicones ; les polyméthylphényl siloxanes; les huiles fluorées éventuellement partiellement hydrocarbonées et/ou siliconées ; et leurs mélanges.

10. Utilisation selon l'une quelconque des revendications précédentes, caractérisée en ce la composition comporte au moins une cire choisie parmi les cires hydrocarbonées, fluorées, siliconées et leurs mélanges.

11. Utilisation selon la revendication 10, caractérisée en ce que la cire est présente à raison de 1 à 50 % du poids total de la composition, de préférence 10 à 30 %.

12. Utilisation selon l'une des revendications précédentes, caractérisée en ce que la composition contient une phase particulaire.

13. Utilisation selon la revendication précédente, caractérisée en ce que la charge particulaire est présente à raison de 0 à 35 % du poids total de la composition, de préférence 5 à 25 %.

14. Utilisation selon l'une des revendications précédentes, caractérisée en ce que la composition se présente sous la forme de stick ou bâton, de pâte souple ou coulée, ou d'une crème ou d'un gel.

15. Utilisation selon l'une des revendications précédentes, caractérisée en ce que la composition contient, en outre, au moins un actif cosmétique ou dermatologique.

16. Utilisation selon l'une des revendications 2 à 15, caractérisée en ce que la composition se présente sous forme d'une composition de fond de teint, de fard à joues ou à paupières, d'anti-cernes ou de rouge à lèvres, dans une base de soin ou une base fixante pour les lèvres, d'un produit dermatologique ou de soin de la peau ou d'une composition solaire.

17. Utilisation selon l'une des revendications précédentes, caractérisée en ce que la composition est un rouge à lèvres ou un fond de teint anhydre contenant au moins une cire et une charge particulaire.

18. Procédé cosmétique pour limiter et/ou empêcher la migration d'une composition de maquillage ou de soin de la peau ou des lèvres, consistant à introduire dans la composition au moins un organopolysiloxane élastomérique solide au moins partiellement réticulé.

## Claims

1. Cosmetic use of a solid, at least partially crosslinked, elastomeric organopolysiloxane, as gelling agent, in a make-up or care composition for a region of human skin or lips, in order to reduce the migration of the said composition around the said region or the lips.

2. Use of a solid, at least partially crosslinked, elastomeric organopolysiloxane, as gelling agent, for the manufacture of a care or treatment composition for a region of human skin or lips, in order to reduce the migration of the said composition around the said region or the lips.

3. Use according to Claim 1 or 2, characterized in that the elastomeric organopolysiloxane is obtained by addition reaction and crosslinking, in the presence of a catalyst, of at least:
(a) an organopolysiloxane having at least two C₂-C₆ lower alkenyl groups per molecule; and
(b) an organopolysiloxane having at least two hydrogen atoms linked to a silicon atom per molecule.

4. Use according to any one of the preceding claims, characterized in that the organopolysiloxane is chosen from:
- i) organopolysiloxanes comprising R₂SiO and RSiO_{1.5} units and optionally R₃SiO_{0.5} and/or SiO₂ units in which the radicals R, independently of each other, represent a hydrogen, an alkyl, an aryl, an unsaturated aliphatic group, the weight ratio of the units R₂SiO to the units RSiO_{1.5} ranging from 1/1 to 30/1;
- ii) organopolysiloxanes which are insoluble and swellable in silicone oil, obtained by addition of an organohydrogenopolysiloxane (1) and of an organopolysiloxane (2) having unsaturated aliphatic groups such that the amount of hydrogen or of unsaturated aliphatic groups in (1) and (2) respectively is between 1 and 20 mol% when the organopolysiloxane is non-cyclic and between 1 and 50 mol% when the organopolysiloxane is cyclic.

5. Use according to one of the preceding claims, characterized in that the solid elastomeric organopolysiloxane is present in the elastomeric organopolysiloxane/fatty phase mixture in order to form a homogeneous gel at a concentration ranging from 0.3 to 40% by weight.

6. Use according to one of the preceding claims, characterized in that the composition contains at least one oil.

7. Use according to Claim 6, characterized in that the oil is a non-volatile oil.

8. Use according to Claim 6 or 7, characterized in that the oil is present in a proportion of 5 to 80%, preferably 30 to 50%, of the total weight of the composition.

9. Use according to any one of the preceding claims, characterized in that the oil is chosen from hydrocarbon oils of animal or plant origin; oils of formula R⁹COOR¹⁰ in which R⁹ represents a higher fatty acid residue containing from 7 to 19 carbon atoms and R¹⁰ represents a branched hydrocarbon chain containing from 3 to 20 carbon atoms; linear or branched hydrocarbons of mineral or synthetic origin; fatty alcohols; synthetic esters and ethers; linear polymethylsiloxanes which are liquid or pasty at room temperature; phenyldimethicones; phenyltrimethicones; polymethylphenylsiloxanes; fluoro oils which are optionally partially hydrocarbonated and/or siliconated; and mixtures thereof.

10. Use according to any one of the preceding claims, characterized in that the composition contains at least one wax chosen from hydrocarbon, fluoro and silicone waxes and mixtures thereof.

11. Use according to Claim 10, characterized in that the wax is present in a proportion of 1 to 50% of the total weight of the composition, preferably 10 to 30%.

12. Use according to one of the preceding claims, characterized in that the composition contains a particulate phase.

13. Use according to the preceding claim, characterized in that the particulate filler is present in a proportion of 0 to 35% of the total weight of the composition, preferably 5 to 25%.

14. Use according to one of the preceding claims, characterized in that the composition is in the form of a stick or tube, of supple or cast paste, or of a cream or a gel.

15. Use according to one of the preceding claims, characterized in that the composition also contains at least one cosmetic or dermatological active agent.

16. Use according to one of Claims 2 to 15, characterized in that the composition is in the form of a foundation, a blusher, an eyeshadow, a concealer product or a lipstick, in a care base or a fixing base for the lips, a dermatological or skincare product or an antisun composition.

17. Use according to one of the preceding claims, characterized in that the composition is an anhydrous lipstick or foundation containing at least one wax and a particulate filler.

18. Cosmetic process for limiting and/or preventing the migration of a make-up or care composition for the skin or the lips, the process consisting in introducing at least one solid, at least partially crosslinked, elastomeric organopolysiloxane into the composition.

## Patentansprüche

1. Kosmetische Verwendung eines mindestens zum Teil vernetzten festen Organopolysiloxans vom Elastomertyp als Gelbildner in einer Zusammensetzung zum Schminken oder zur Pflege einer Hautpartie oder der Lippen von Personen, um die Migration der Zusammensetzung um die Hautpartie oder die Lippen zu vermindern.

2. Verwendung eines mindestens zum Teil vernetzten festen Organopolysiloxans vom Elastomertyp als Gelbildner zur Herstellung einer Zusammensetzung zur Pflege oder zur Behandlung einer Hautpartie oder der Lippen von Personen, um die Migration der Zusammensetzung um die Hautpartie oder die Lippen zu vermindern.

3. Verwendung nach Anspruch 1 oder 2, dadurch gekennzeichnet, daß das Organopolysiloxan vom Elastomertyp durch Addition und Vernetzung hergestellt wird, wobei mindestens:
- (a) ein Organopolysiloxan, das pro Molekül mindestens zwei niedere C₂₋₆-Alkenylgruppen aufweist, und
- (b) ein Organopolysiloxan, das pro Molekül mindestens zwei Wasserstoffatome aufweist, die an ein Siliciumatom gebunden sind,
in Gegenwart eines Katalysators verwendet werden.

4. Verwendung nach einem der vorhergehenden Ansprüche, dadurch gekennzeichnet, daß das Organopolysiloxan ausgewählt ist unter:
- (i) Organopolysiloxanen, die Einheiten R₂SiO und RSiO_{1,5} und gegebenenfalls Einheiten R₃SiO_{0,5} und/oder SiO₂ aufweisen, worin die Gruppen R unabhängig voneinander Wasserstoff, eine Alkylgruppe, eine Arylgruppe oder eine ungesättigte aliphatische Gruppe bedeuten und worin das Gewichtsverhältnis der Einheiten R₂SiO zu den Einheiten RSiO_{1,5} im Bereich von 1/1 bis 30/1 liegt, und
- (ii) Organopolysiloxanen, die in dem Siliconöl unlöslich und quellfähig sind und die durch Addition eines Organohydrogenopolysiloxans (1) und eines Organopolysiloxans (2) mit ungesättigten aliphatischen Gruppen so hergestellt werden, daß die Wasserstoffmenge oder die Menge ungesättigter aliphatischer Gruppen in (1) bzw. (2) im Bereich von 1 bis 20 Mol-% liegt, wenn das Organopolysiloxan nicht cyclisch vorliegt, und im Bereich von 1 bis 50 Mol-%, wenn das Organopolysiloxan cyclisch vorliegt.

5. Verwendung nach einem der vorhergehenden Ansprüche, dadurch gekennzeichnet, daß das feste Organopolysiloxan vom Elastomertyp in dem Gemisch Organopolysiloxan vom Elastomertyp/Fettphase zur Bildung eines homogenen Gels in einer Konzentration von 0,3 bis 40 Gew.-% vorliegt.

6. Verwendung nach einem der vorhergehenden Ansprüche, dadurch gekennzeichnet, daß die Zusammensetzung mindestens ein Öl enthält.

7. Verwendung nach Anspruch 6, dadurch gekennzeichnet, daß das Öl ein nicht flüchtiges Öl ist.

8. Verwendung nach Anspruch 6 oder 7, dadurch gekennzeichnet, daß das Öl in einem Mengenanteil von 5 bis 80 % und vorzugsweise von 30 bis 50 %, bezogen auf das Gesamtgewicht der Zusammensetzung, vorliegt.

9. Verwendung nach einem der vorhergehenden Ansprüche, dadurch gekennzeichnet, daß das Öl ausgewählt ist unter Kohlenwasserstoffölen tierischen und pflanzlichen Ursprungs; Ölen der Formel R⁹COOR¹⁰, worin R⁹ den Rest einer höheren Fettsäure bedeutet, die 7 bis 19 Kohlenstoffatome aufweist, und worin R¹⁰ eine verzweigte Kohlenwasserstoffkette bedeutet, die 3 bis 20 Kohlenstoffatome enthält; geradkettigen oder verzweigten Kohlenwasserstoffen mineralischen oder synthetischen Ursprungs; Fettalkoholen; synthetischen Estern und Ethern; geradkettigen Polymethylsiloxanen, die bei Umgebungstemperatur flüssig oder pastös sind; Phenyldimeticonen; Phenyltrimeticonen; Polymethylphenylsiloxanen; fluorierten Ölen, die gegebenenfalls zum Teil kohlenwasserstoffhaltig und/oder siliconhaltig sind und den Gemischen dieser Verbindungen.

10. Verwendung nach einem der vorhergehenden Ansprüche, dadurch gekennzeichnet, daß die Zusammensetzung mindestens ein Wachs enthält, das unter kohlenwasserstoffhaltigen, fluorhaltigen und siliconhaltigen Wachsen und den Gemischen dieser Wachse ausgewählt ist.

11. Verwendung nach Anspruch 10, dadurch gekennzeichnet, daß das Wachs in einem Mengenanteil von 1 bis 50 % und vorzugsweise von 10 bis 30 %, bezogen auf das Gesamtgewicht der Zusammensetzung, vorliegt.

12. Verwendung nach einem der vorhergehenden Ansprüche, dadurch gekennzeichnet, daß die Zusammensetzung eine Partikelphase enthält.

13. Verwendung nach dem vorhergehenden Anspruch, dadurch gekennzeichnet, daß die Partikelphase in einem Mengenanteil von 0 bis 35 % und vorzugsweise von 5 bis 25 %, bezogen auf das Gesamtgewicht der Zusammensetzung, vorliegt.

14. Verwendung nach einem der vorhergehenden Ansprüche, dadurch gekennzeichnet, daß die Zusammensetzung in Form eines Sticks oder Stifts, als weiche oder gegossene Paste, als Creme oder als Gel vorliegt.

15. Verwendung nach einem der vorhergehenden Ansprüche, dadurch gekennzeichnet, daß die Zusammensetzung ferner mindestens einen kosmetischen oder dermatologischen Wirkstoff enthält.

16. Verwendung nach einem der Ansprüche 2 bis 15, dadurch gekennzeichnet, daß die Zusammensetzung in Form einer Zusammensetzung für Make-up, Wangenrouge, Lidschatten, Abdeckmittel für Augenringe oder Lippenstift, in einer Pflegegrundmasse oder fixierenden Grundmasse für die Lippen, als dermatologisches Produkt, als Produkt zur Pflege der Haut oder als Sonnenschutzzusammensetzung vorliegt.

17. Verwendung nach einem der vorhergehenden Ansprüche, dadurch gekennzeichnet, daß die Zusammensetzung ein wasserfreier Lippenstift oder ein wasserfreies Make-up ist, die mindestens ein Wachs und mindestens eine Partikelphase enthält.

18. Kosmetisches Verfahren, um die Migration einer Zusammensetzung zum Schminken oder zur Pflege der Haut oder der Lippen zu vermindern und/oder zu unterbinden, das darin besteht, in die Zusammensetzung mindestens ein festes Organopolysiloxan vom Elastomertyp einzubringen, das mindestens zum Teil vernetzt ist.
